# NEUE EUROPÄISCHE PATENTSCHRIFT

(11) **EP 0 938 369 B2**
(45) Veröffentlichungstag und Bekanntmachung des Hinweises auf die Entscheidung über den Einspruch: **04.10.2006**
(45) Hinweis auf die Patenterteilung: 12.11.2003
(21) Anmeldenummer: 97919000.6
(22) Anmeldetag: 25.08.1997
(51) Int. Cl.: B01F 17/00, A61K 8/30

(54) **EMULGATORMISCHUNGEN UND DEREN VERWENDUNG ZUR HERSTELLUNG VON PIT-EMULSIONEN**
EMULSIFIER MIXTURES AND THEIR USE FOR PREPARING PIT EMULSIONS
MELANGES EMULSIFIANTS ET LEUR EMULSION POUR LA PREPARATION D'EMULSIONS PIT

(30) Priorität: 02.09.1996 DE 19635553
(43) Veröffentlichungstag der Anmeldung: 01.09.1999
(73) Patentinhaber: Cognis IP Management GmbH, 40589 Düsseldorf (DE)
(72) Erfinder: WADLE, Armin, F-91590 Cerny (FR); ANSMANN, Achim, D-40699 Erkrath (DE); TESMANN, Holger, D-41363 Jüchen (DE); GANTKE, Karl-Heinz, D-41065 Mönchengladbach (DE); BEHLER, Ansgar, D-46240 Bottrop (DE); GUCKENBIEHL, Bernhard, D-40591 Düsseldorf (DE)
(86) Internationale Anmeldenummer: PCT/EP1997/004621
(87) Internationale Veröffentlichungsnummer: WO 1998/009721

(56) Entgegenhaltungen:
- EP-A- 0 345 586
- EP-A- 0 617 955
- WO-A-93/11865
- WO-A-96/28131
- DE-A- 4 010 393
- GB-A- 2 278 780
- Auszug aus dem Lexicon der Hilfsstoffe, Stichwort Glycerylisostearat

## Beschreibung

### Gebiet der Erfindung

Die Erfindung betrifft die Verwendung von Emulgatormischungen auf Basis ausgewählter nichtionischerTenside zur Herstellung von PIT-Emulsionen.

### Stand der Technik

Zur Herstellung feinteiliger Emulsionen über die Phaseninversionstemperatur (PIT-) Methode werden üblicherweise Mischungen aus hydrophilen Emulgatoren und hydrophoben Co-Emulgatoren eingesetzt (vgl. **DE-A1 38** 19193, **DE-A1 4010 393, DE-A1 4140 562, DE-A1 43 18 171, DE-A1 43 37 041**, **DE-A1 44 11 557** [Henkel]). Nach dem Stand der Technik gilt, dass für die PIT Emulgiertechnologie der Anteil des Emulgators und des Co-Emulgators jeweils an den Ölcharakter- ausgedrückt über die sogenannte ACN-Zahl - angepasst werden muss. Es sind demzufolge keine EmulgatorlCo-Emulgator-Mischungen bekannt, die in der Lage wären, verschiedene Ölkörper unter gleichen Temperaturbedingungen nach der PIT-Methode zu emulgieren. Es liegt auf der Hand, dass solche Mischungen die Herstellung von stabilen Emulsionen erheblich erleichtern würden. Die Aufgabe der Erfindung hat demzufolge darin bestanden, Emulgatormischungen zur Herstellung von PIT-Emulsionen zur Verfügung zu stellen, die frei von den geschilderten Nachteilen sind.

### Beschreibung der Erfindung

Gegenstand der Erfindung ist die Verwendung von Emulgatormischungen, enthaltend
(a) Fettsäureethoxylate und
(b) Partialglyceride zur Herstellung von PIT-Emulsionen, wobei man die Komponenten (a) und (b) in Gewichsverhältnis 40:60 bis 60:40 einsetzt.

Überraschenderweise wurde gefunden, daß diese Mischungen es gestatten, PIT-Emulsionen bei gegebener Phaseninversionstemperatur unabhängig von der ACN-Zahl, d.h. von der Polarität der Ölkörper, herzustellen. Die Mischungen sind daher geeignet, eine breite Palette von Ölkörpern unabhängig vom Ölcharakter unter gleichen Bedingungen zu emulgieren.

### Fettsäureethoxylate

Fettsäureethoxylate, die als Emulgatorkomponente (a) in Betracht kommen, folgen vorzugsweise der Formel (I),

**R**^{**1**}**COO(CH**_{**2**}**CH**_{**2**}**O)**_{**n**}**H** (I)

in der R¹CO für einen linearen oder verzweigten Acylrest mit 12 bis 22 Kohlenstoffatomen und n für Zahlen von 5 bis 50 und vorzugsweise 15 bis 35 steht. Typische Beispiele sind Anlagerungsprodukte von 20 bis 30 Mol Ethylenoxid an Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen oder bei der Reduktion von Aldehyden aus der Roelen'schen Oxosynthese anfallen. Vorzugsweise werden Anlagerungsprodukte von 20 bis 30 Mol Ethylenoxid an Fettsäuren mit 16 bis 18 Kohlenstoffatomen eingesetzt.

### Partialglyceride

Partialglyceride, die als Emulgatorkomponente (b) in Betracht kommen, folgen vorzugsweise der Formel (II), 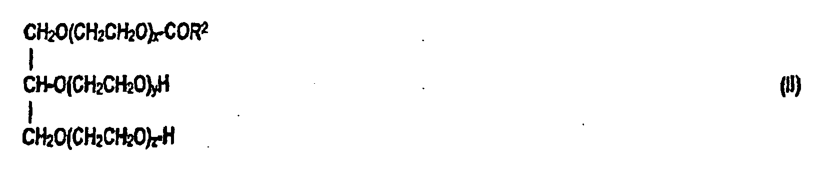 in der R²CO für einen linearen oder verzweigten Acylrest mit 12 bis 22 Kohlenstoffatomen und x, y und z in Summe für 0 oder für Zahlen von 1 bis 50, vorzugsweise 15 bis 35 steht. Typische Beispiele fürim Sinne der Erfindung geeignete Partialglyceride sind Laurinsäuremonoglycerid, Kokosfettsäuremonoglycerid, Palmitinsäuremonoglycerid, Stearinsäuremonoglycerid, Isostearinsäuremonoglycerid, Ölsäurebis 30 Mol Ethylenoxid. Vorzugsweise werden Monoglyceride bzw. technische Mono/Diglyceridgernische mit überwiegendem Monoglyceridanteil der Formel (II) eingesetzt, in der R²CO für einen linearen Acylrest mit 16 bis 18 Kohlenstoffatomen steht.

### Gewerbliche Anwendbarkeit

Mit Hilfe der Emulgatormischungen lassen sich stabile PIT-Emulsionen mit Ölkörpern unterschiedlichster Po-<SHY>unterschiedlichster Polarität herstellen. Die Erfindung betrifft daher die Verwendung der Emulgatormischungen zur Herstellung von PIT-Emulsionen, wobei man die Mischungen in der Regel in Mengen von 1 bis 10, vorzugsweise 3 bis 8 Gew.-% - bezogen auf die Emulsionen - einsetzt.

### Olkörper

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₀-Fettsäuren mit linearen C₆-C₂₀-Fettalkoholen, Ester von verzweigten C₆-C₁₃-Carbonsäuren mit linearen C₆-C₂₀-Fettalkoholen, Ester von linearen C₆-C₁₈-Fettsäuren mit verzweigten Alkoholen, insbesondere 2-Ethylhexanol, Ester von linearen und/oder verzweigten Fettsäuren mit mehrwertigen Alkoholen (wie z.B. Dimerdiol oder Trimertriol) und/oder Guerbetalkoholen, Triglyceride auf Basis C₆-C₁₀-Fettsäuren, pflanzliche Öle, verzweigte primäre Alkohole, substituierte Cyclohexane, Guerbetcarbonate, Dialkylether und/oder allphatische bzw. naphthenische Kohlenwasserstoffe in Betracht, die man üblicherweise in Mengen von 10 bis 50, vor zugsweise 15 bis 35 Gew.-% - bezogen auf die Emulsionen - einsetzt.

Die PIT-Emulsionen können für eine Vielzahl von Anwendungszwecken, vorzugsweise aber für die Herstellung von Haut- und Haarpflegemittel eingesetzt werden, wie beispielsweise Haarshampoos, Haarlotionen, Schaumbäder, Cremes, Lotionen oder Salben. Sie können femer als weitere Hilfs- und Zusatzstoffe Tenside Co-Emulgatoren, Überfettungsmittel, Stabilisatoren, Wachse, Konsistenzgeber, Verdictcungsmittel, Kationpolymere, Siliconverbindungen, biogene Wirkstoffe, Antischuppenmittel, Filmbildner, Konservierungsmittel, Hydrotrope, Solubilisatoren, UV-Adsorber, Farb- und Duftstoffe und dergleichen enthalten.

Typische Beispiele für geeignete **Tenside** sind Fettalkoholpolyglycolethersulfate, Monoglyceridsulfate, Mono- und/oder Dialkylsulfosuccinate; Fettsäureisethionate, Fettsäuresarco-sinate, Fettsäuretauride, Ethercarbonsäuren, Alkyloligoglucoside, Fettsäureglucamide, Alkylamidobetaine und/oder vorzugsweise pflanzliche Proteinfettsäurekondensate.

Als **Co-Emulgatoren** kommen beispielsweise nichtionogene Tenside aus mindestens einer der folgenden Gruppen in Frage:
(b1) Anlagerungsprodukte von 2 bis 30 Mol Ethylenoxid und/ oder 0 bis 5 Mol Propylenoxid an lineare Fettalkohole mit 8 bis 22 C-Atomen und an Alkylphenole mit 8 bis 15 C-Atomen in der Alkylgruppe;
(b2) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(b3) Alkylmono- und -oligoglycoside mit 8 bis 22 Kohlenstoffatomen im Alkylrest und deren ethoxylierte Analoga;
(b4) Anlagerungsprodukte von 15 bis 60 Mol Ethylenoxid an Ricinusöl und/oder gehärtetes Ricinusöl;
(b5) Polyol- und insbesondere Polyglycerinester wie z.B. Polyglycerinpolyricinoleat oder Polyglycerinpoly-12-hydroxystearat. Ebenfalls geeignet sind Gemische von Verbindungen aus mehreren dieser Substanzklassen;
(b6) Anlagerungsprodukte von 2 bis 15 Mol Ethylenoxid an Ricinusöl und/oder gehärtetes Ricinusöl;
(b7) Partialester auf Basis linearer, verzweigter, ungesättigter bzw. gesättigter C_{12/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit) sowie Polyglucoside (z.B. Cellulose);
(b8) Trialkylphosphate;
(b9) Wollwachsalkohole;
(b10) Polysiloxan-Polyalkyl-Polyether-Copolymere bzw. entsprechende Derivate;
(b11) Mischester aus Pentaerythrit, Fettsäuren, Citronensäure und Fettalkohol gemäß **DE.PS 11 65 574** sowie
(b12) Polyalkylenglycole.

Die Anlagerungsprodukte von Ethylenoxid und/oder von Propylenoxid an Fettalkohole, Alkylphenole, Glycerinmono- und -diestersowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht.

C_{8/18}-Alkylmono- und -oligoglycoside, ihre Herstellung und ihre Verwendung als oberflächenaktive Stoffe sind beispielsweise aus **US 3,839,318, US 3,707,535, US 3,547,828, DE-OS 19 43 689, DE-OS 20 36 472** und **DE-A1** 30 **01 064** sowie **EP-A 0 077167** bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumgiycinat, und 2-Alkyl-3-carboxylmethyl-3-hydroxyethylimidazoline mit jeweils 8 bis 18 C-Atomen in der Alkyloder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine. N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}Acylsarcosin. Neben den ampholytischen kommen auch quartäre Emulgatoren in Betracht, wobei solche vom Typ der Esterquats, vorzugsweise methytquatemierte Difettsäuretriethanolaminester-Salze, besonders bevorzugt sind.

Als **Überfettungsmittel** können Substanzen wie beispielsweise Lanolin und Lecithin sowie polyethoxylierte oder acylierte Lanolin- und Lecithinderivate, Polyolfettsäureester und Fettsäurealkanolamide verwendet werden, wobei die letzteren gleichzeitig als Schaumstabilisatoren dienen. Als **Konsistenzgeber** kommen in erster Linie Fettalkohole mit 12 bis 22 und vorzugsweise 16 bis 18 Kohlenstoffatomen in Betracht. Bevorzugt ist eine Kombination dieser Stoffe mit Alkyloligoglucosiden und/oder Fettsäure-N-methyl-glucamiden gleicher Kettenlänge undloder Polyglycerinpoly-12-hydroxystearaten. Geeignete **Verdickungsmittel** sind beispielsweise Potysaccharide, insbesondere Xanthan-Gum, Guar-Guar, Agar-Agar, Alginate und Tylosen, Carboxymethylcellulose und Hydroxyethylcellulose, femer höhermolekulare Polyethylenglycolmono- und -diester von Fettsäuren, Polyacrylate, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, kationische Stärke, Copolymere von Diallylammoniumsalzen und Acrylamiden, quaternierte VinylpyrrolidonNinylimidazol-Polymere wie z.B. Luviquat® (BASF AG, Ludwigshafenl FRG), Kondensationsprodukte von Polyglycolen und Aminen, quatemierte Kollagenpolypeptide wie beispielsweise Lauryldimonium hydroxypropyl hydrolyzed collagen (Lamequat®L, Grünau GmbH), quatemierte Weizenpolypeptide, Polyethylenimin, kationische Siliconpolymere wie z.B. Amidomethicone oder Dow Coming, Dow Corning Co./US, Copolymere der Adipinsäure und Dimethylaminohydroxypropyldiethylentrimamin (Cartaretine®, Sandoz/CH), Polyaminopolyamide wie z.B. beschrieben in der **FR-A 22 52 840** sowie deren vemetzte wasserlöslichen Polymere, kationische Chitinderivate wie beispielsweise quaterniertes Chitosan, gegebenenfalls mikrokristallin verteilt, Kondensationsprodukte aus Dihalogenalkylen wie z.B. Dibrombutan mit Bisdialkylaminen wie z. B. Bis-Dimethylamino-1,3-propan, kationischer Guar-Gum wie z.B. Jaguar® CBS, Jaguar® C-17, Jaguar® C-16 der Celanese/US, quaternierte Ammoniumsalz-Polymere wie z.B. Mirapol® A-15, Mirapol® AD-1, Mirapol® AZ-1 der Miranol/US.

Geeignete Siliconverbindungen sind beispielsweise Dimethylpolysiloxane, Methyl-phenylpolysiloxane, cydische Silicone sowie amino-, fettsäure-, alkohol-, polyether-, epoxy-, fluor- und/oder alkylmodifizierte Siliconverbindungen, die bei Raumtemperatur sowohl flüssig als auch harzförmig vorliegen können. Typische Beispiele für Fette sind Glyceride, als **Wachse** kommen u.a. Bienenwachs, Paraffinwachs oder Mikrowachse gegebenenfalls in Kombination mit hydrophilen Wachsen, z.B. Cetylstearylalkohol in Frage. Als Perlglanzwachse können insbesondere Mono- und Difettsäureestervon Polyalkylenglycolen, Partialglyceride oder Ester von Fettalkoholen mit mehrwertigen Carbonsäuren bzw. Hydroxycarbonsäuren verwendet werden. Als **Stabilisatoren** können Metaltsalze von Fettsäuren wie z.B. Magnesium-, Aluminium- undloder Zinkstearat eingesetzt werden. Unter **biogenen Wirkstoffen** sind beispielsweise Bisabolol, Allantoin, Phytantriol, Panthenol, AHA-Säuren, Pflanzenextrakte und Vitaminkomplexe zu verstehen. Als **Antischuppenmittel** können Climbazol, Octopirox und Zinkpyrethion eingesetzt werden. Gebräuchliche **Filmbildner** sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quatemäre Cellulose-Derivate, Kollagen, Hyaluronsäure bzw. deren Salze und ähnliche Verbindungen. Zur Verbesserung des Fließverhaltens können femer **Hydrotrope** wie beispielsweise Ethanol, Isopropylalkohol, Propylenglycol oder Glucose eingesetztwerden. Als **Konservierungsmittel** eignen sich beispielsweise Phenoxyethanol, Formaldehydlösung, Parabene, Pentandiol oder Sorbinsäure. Als Farb**stoffe** können die für kosmetische Zwecke geeigneten und zugelassenen Substanzen verwendet werden, wie sie beispielsweise in der Publikation **"Kosmetische Färbemittel" der Farbstoffkommission der** Deutschen For**schungsgemeinschaft, Verlag Chemie, Weinheim, 1984, S.81-106** zusammengestellt sind. Diese Farbstoffe werden üblicherweise in Konzentrationen von 0,001 bis 0,1 Gew.%, bezogen auf die gesamte Mischung, eingesetzt

Der Gesamtanteil der Hilfs- und Zusatzstoffe kann 1 bis 50, vorzugsweise 5 bis 40 Gew.-% - bezogen auf die Emulsionen - betragen.

### Beispiele

Es wurden unter Verwendung der Emulgatormischungen (R1 bis R3) und einer Vergleichsmischung (R4) Emulsionen auf Basis von Ölkörpem unterschiedlicher Polarität nach der PIT-Methode hergestellt. Die Emulsionen wurden 4 Wochen bei 40°C gelagert und anschließend visuell auf ihre Stabilität geprüft. Die Ergebnisse sind in Tabelle 1 zusammengefaßt.

**Tabelle 1;**

| **Stabilität von Emulsionen (Mengenangaben als Gew.-%)** | | | | |
|---|---|---|---|---|
| | **R1** | **R2** | **R3** | **R4** |
| Palmitinstearinsäure+30 EO | 3,3 | 3,3 | 1,7 | - |
| Cetearylalkohof+30 EO | - | - | - | 3,3 |
| Glyceryl Stearate | 3,3 | 3,3 | 1,7 | 3,3 |
| Cetiol LC | 20,0 | - | - | - |
| OleylOleate | - | 20,0 | - | 20,0 |
| Capric/Caprylic Triglycerides | - | - | 5,0 | - |
| Paraffinöl, dünnflüssig | - | - | 5,0 | - |
| Wasser | ad 100 | | | |
| ***PIT[°C]*** | 85 | 85 | 85 | - |
| ***Stabilität nach 3 Wochen Lagerung (40°*C)** | stabil | stabil | stabil | instabil |

Man erkennt, daß bei Austausch der Fettsäureethoxylate gegen Fettalkoholethoxylate keine stabilen PIT-Emulsionen mehr entstehen.

## Patentansprüche

1. Verwendung von Emulgatormischungen, enthaltend
(a) Fettsäureethoxylate und
(b) Partialglyceride
zur Herstellung von PIT-Emulsionen, wobei man die Komponenten (a) und (b) im Gewichtsverhältnis 40:60 bis 60:40 einsetzt.

2. Verwendung nach Anspruch 1, **dadurch gekennzeichnet, daß** man Fettsäureethoxylate der Formel (I) einsetzt,
R¹COO(CH₂CH₂O)ₙH (I)
in der R¹CO für einen linearen oder verzweigten Acylrest mit 12 bis 22 Kohlenstoffatomen und n für Zahlen von 5 bis 50 steht.

3. Verwendung nach den Ansprüchen 1 und/oder 2, **dadurch gekennzeichnet, daß** man Partialglyceride der Formel **(II)** einsetzt, 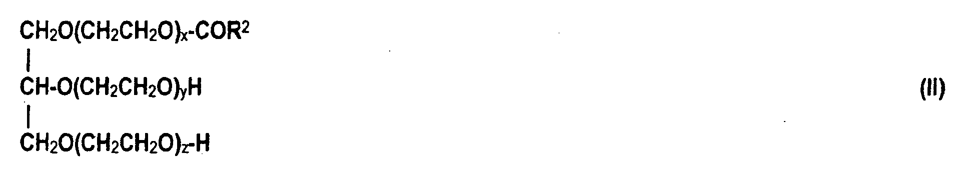 in der R²CO für einen linearen oder verzweigten Acylrest mit 12 bis 22 Kohlenstoffatomen und x, y und z in Summe für 0 oder für Zahlen von 5 bis 50, vorzugsweise 15 bis 35 steht.

4. Verwendung nach mindestens einem der Ansprüche 1 bis 3, **dadurch gekennzeichnet; daß** man die Emulgatormischungen in Mengen von 1 bis 10 Gew.-% - bezogen auf die Emulsionen - einsetzt.

5. Verwendung nach mindestens einem der Ansprüche 1 bis 4, **dadurch gekennzeichnet, daß** man zur Herstellung der PIT-Emulsionen Ölkörper einsetzt, die ausgewählt sind aus der Gruppe, die gebildet wird von Guerbetalkoholen auf Basis von Fettalkoholen mit 6 bis 18 Kohlenstoffatomen, Estern von linearen C₆-C₂₀-Fettsäuren mit linearen C₆-C₂₀-Fettalkoholen, Estern von verzweigten C₆-C₁₃-Carbonsäuren mit linearen C₆-C₂₀-Fettalkoholen, Estern von linearen C₆-C₁₈-Fettsäuren mit verzweigten Alkoholen, Estern von linearen und/oder verzweigten Fettsäuren mit mehrwertigen Alkoholen und/oder Guerbetalkoholen, Triglyceriden auf Basis C₆-C₁₀Fettsäuren, pflanzlichen Ölen, verzweigten primären Alkoholen, substituierten Cyclohexanen, Guerbetcarbonaten, Dialkylethen und/oder aliphatischen bzw. naphthenischen Kohlenwasserstoffen.

6. Verwendung nach Anspruch 5, **dadurch gekennzeichnet, daß** man die Ölkörper in Mengen von 10 bis 50 Gew.-% - bezogen auf die Emulsionen - einsetzt.

## Claims

1. The use of emulsifier mixtures containing
(a) fatty acid ethoxylates and
(b) partial glycerides
for the production of PIT emulsions, components (a) and (b) being used in a ratio by weight of 40:60 to 60:40.

2. The use claimed in claim 1, **characterized in that** fatty acid ethoxylates corresponding to formula (I):
**R**^{**1**}**COO(CH**_{**2**}**CH**_{**2**}**O)**_{**n**}**H** (I)
in which R¹CO is a linear or branched acyl group containing 12 to 22 carbon atoms and n is a number of 5 to 50,
are used.

3. The use claimed in claims 1 and/or 2, **characterized in that** partial glycerides corresponding to formula (II): 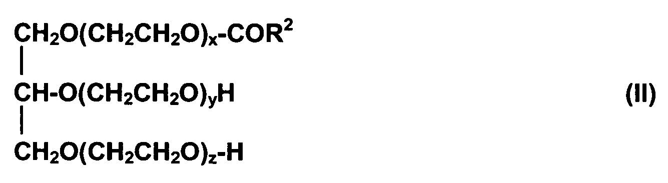 in which R²CO is a linear or branched acyl group containing 12 to 22 carbon atoms and x, y and z together stand for 0 or for numbers of 5 to 50 and preferably 15 to 35,
are used.

4. The use claimed in at least one of claims 1 to 3, **characterized in that** the emulsifier mixtures are used in quantities of 1 to 10% by weight, based on the emulsions.

5. The use claimed in at least one of claims 1 to 4, **characterized in that** oil components selected from the group consisting of Guerbet alcohols based on fatty alcohols containing 6 to 18 carbon atoms, esters of linear C₆₋₂₀ fatty acids with linear C₆₋₂₀ fatty alcohols, esters of branched C₆₋₁₃ carboxylic acids with linear C₆₋₂₀ fatty alcohols, esters of linear C₆₋₁₈ fatty acids with branched alcohols, esters of linear and/or branched fatty acids with polyhydric alcohols and/or Guerbet alcohols, triglycerides based on C₆₋₁₀ fatty acids, vegetable oils, branched primary alcohols, substituted cyclohexanes, Guerbet carbonates, dialkyl ethers and/or aliphatic or naphthenic hydrocarbons are used for the production of the PIT emulsions.

6. The use claimed in claim 5, **characterized in that** the oil components are used in quantities of 10 to 50% by weight, based on the emulsions.

## Revendications

1. Utilisation de mélanges d'agents émulsionnants contenant
a) des éthoxylates d'acide gras et
b) des glycérides partiels
en vue de la production d'émulsions de type PIT, avec un rapport en poids des composants a) et b) de 40 :60 à 60 :40.

2. Utilisation selon la revendication 1,
**caractérisée en ce qu'**
on met en oeuvre des éthoxylates d'acide gras de formule (I)
R¹COO(CH₂CH₂O)ₙH (I)
dans laquelle R¹CO représente un reste acyle linéaire ou ramifié ayant de 12 à 22 atomes de carbone et n représente des nombres allant de 5 à 50.

3. Utilisation selon les revendications 1 et/ou 2,
**caractérisée en ce qu'**
on met en oeuvre des glycérides partiels de formule II 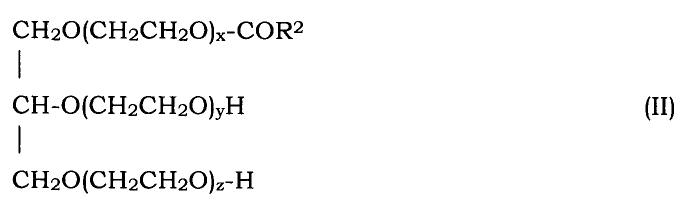 dans laquelle R²CO représente un reste acyle linéaire ou ramifié ayant de 12 à 22 atomes de carbone et x, y et z représentent globalement 0 ou des nombres allant de 5 à 50, de préférence de 15 à 35.

4. Utilisation selon au moins une des revendications 1 à 3,
**caractérisée en ce qu'**
on met en oeuvre les mélanges d'agents émulsionnants en quantités allant de 1 à 10 % en poids - rapporté aux émulsions.

5. Utilisation selon au moins une des revendications 1 à 4,
**caractérisée en ce qu'**
on met en oeuvre en outre en vue de la production des émulsions du type PIT, des composés huileux choisis dans le groupe formé des alcools de Guerbet à base d'alcool gras ayant de 6 à 18 atomes de carbone, des esters d'acide gras linéaires en C₆-C₂₀ avec des alcools gras linéaires en C₆-C₂₀, des esters d'acides carboxyliques ramifiés en C₆-C₁₃, avec des alcools gras linéaires en C₆-C₂₀, des esters d'acides gras linéaires en C₆₋C₁₈ avec des alcools ramifiés, des esters d'acides gras linéaires et/ou ramifiés avec des alcools plurifonctionnels et/ou des alcools de Guerbet, des triglycérides à base d'acides gras en C₆-C₁₀, des huiles végétales, des alcools primaires ramifiés, des cyclohexanes substitués, des carbonates de Guerbet, des éthers dialkyliques et/ou des hydrocarbures aliphatiques ou naphténiques.

6. Utilisation selon la revendication 5,
**caractérisée en ce qu'**
on met en oeuvre les composés huileux en quantités allant de 10 à 50 % en poids - rapporté aux émulsions.
